# EUROPEAN PATENT APPLICATION

(11) **EP 2 287 335 A1**
(43) Date of publication of application: **23.02.2011**
(21) Application number: 09167294.9
(22) Date of filing: 05.08.2009
(51) Int. Cl.: C12Q 1/68

(54) **Highly sensitive rapid isothermal method for the detection of methylation patterns**

(71) Applicant: DiaSorin S.p.A., 13040 Saluggia VC (IT)
(72) Inventor: Shehi, Erlet, 13040 Saluggia (VC) (IT); Adlerstein, Daniel, 13040 Saluggia (VC) (IT); Bonanno, Cinzia, 13040 Saluggia (VC) (IT); Makrigiorgos, Gerassimos Mike, 13040 Saluggia (VC) (IT); Zerilli, Francesco, 13040 Saluggia (VC) (IT)
(74) Representative: Comoglio, Elena

(57) **Abstract**

The current invention refers to a method for detecting methylation patterns of a nucleotide sequence by an improvement of the LAMP (Loop-mediated Isothermal Amplification) amplification method. As non limitative embodiment the invention refers to the simplex or multiplex detection of methylation patters in the CDKN2A, GATA5 and DAPK1 human genes.

SEQUENCE LISTING

<110>  DIASORIN SpA

<120>  HHIGHLY SENSITIVE RAPID ISOTHERMAL METHOD FOR THE DETECTION OF
       METHYLATION PATTERNS

<130>  BE107173

<160>  27

<170>  PatentIn version 3.5

<210>  1
<211>  212
<212>  DNA
<213>  Artificial Sequence

<220>
<223>  synthetic oligonucleotide

<400>  1
tggagtgtga ggaggatagt tggattgagt taatgttggg gattttgttt tttttgtgga      60

ggggatttgg taatttgtag tggtagggtt tggggttggt gtttgggagg gatttgtgtt     120

ttttatttat tttttagttg tgtttttgtt gttgttttgg ttagtttttg gtgttggtgt     180

ttatggttgg tttttgatag tgttttggag gg                                   212


<210>  2
<211>  212
<212>  DNA
<213>  Artificial Sequence

<220>
<223>  synthetic oligonucleotide

<400>  2
cggagtgtga ggaggatagt cggatcgagt taacgtcggg gattttgttt ttttcgcgga      60

ggggattcgg taattcgtag cggtagggtt tggggtcggc gtttgggagg gatttgcgtt     120

ttttatttat tttttagttg tgttttcgtc gtcgtttcgg ttagttttcg gcgttggcgt     180

ttatggtcgg ttttcgatag cgtttcggag gg                                   212


<210>  3
<211>  21
<212>  DNA
<213>  Artificial Sequence

<220>
<223>  synthetic oligonucleotide

<400>  3
ggaggatagt cggatcgagt t                                                21


<210>  4
<211>  19
<212>  DNA
<213>  Artificial Sequence

<220>
<223>  synthetic oligonucleotide

<400>  4
cgctatcgaa aaccgacca                                                    19


<210>  5
<211>  42
<212>  DNA
<213>  Artificial Sequence

<220>
<223>  synthetic oligonucleotide

<400>  5
aacgccgacc ccaaacccta cggattttgt ttttttcgcg ga                          42


<210>  6
<211>  42
<212>  DNA
<213>  Artificial Sequence

<220>
<223>  synthetic oligonucleotide

<400>  6
gggagggatt tgcgtttttt attccaacgc cgaaaactaa cc                          42


<210>  7
<211>  21
<212>  DNA
<213>  Artificial Sequence

<220>
<223>  synthetic oligonucleotide

<400>  7
cgctacgaat taccgaatcc c                                                 21


<210>  8
<211>  21
<212>  DNA
<213>  Artificial Sequence

<220>
<223>  synthetic oligonucleotide

<400>  8
agttgtgttt tcgtcgtcgt t                                                 21


<210>  9
<211>  16
<212>  DNA
<213>  Artificial Sequence

<220>
<223>  synthetic oligonucleotide

<400>  9
tggggcggat cgcgtg                                                       16


<210>  10
<211>  22
<212>  DNA
<213>  Artificial Sequence

<220>
<223>  synthetic oligonucleotide

<400>  10
accgtaacta ttcgatacgt ta                                              22


<210>  11
<211>  37
<212>  DNA
<213>  Artificial Sequence

<220>
<223>  synthetic oligonucleotide

<400>  11
cccgccgccg actccatact acttcggcgg ttgcgga                              37


<210>  12
<211>  40
<212>  DNA
<213>  Artificial Sequence

<220>
<223>  synthetic oligonucleotide

<400>  12
ggagttttcg gttgattggt tggtcccccg cctccaacaa                           40


<210>  13
<211>  20
<212>  DNA
<213>  Artificial Sequence

<220>
<223>  synthetic oligonucleotide

<400>  13
cgctacctac tctcccctc                                                  20


<210>  14
<211>  19
<212>  DNA
<213>  Artificial Sequence

<220>
<223>  synthetic oligonucleotide

<400>  14
gggtcgggta gaggaggtg                                                  19


<210>  15
<211>  18
<212>  DNA
<213>  Artificial Sequence

<220>
<223>  synthetic oligonucleotide

<400>  15
tcgtagggtt agcgttgg                                                   18


<210>  16
<211>  18

<212>  DNA
<213>  Artificial Sequence

<220>
<223>  synthetic oligonucleotide

<400>  16
ctcgaaactc gcacaaac                                                    18


<210>  17
<211>  45
<212>  DNA
<213>  Artificial Sequence

<220>
<223>  synthetic oligonucleotide

<400>  17
taaccgcccc gtatcgtacg tcgtcgtaga gttacgtgat tttgg                      45


<210>  18
<211>  43
<212>  DNA
<213>  Artificial Sequence

<220>
<223>  synthetic oligonucleotide

<400>  18
gagttcggat tagtgtagtt agacggtctc tacgcccgac tcc                        43


<210>  19
<211>  17
<212>  DNA
<213>  Artificial Sequence

<220>
<223>  synthetic oligonucleotide

<400>  19
gccaaacccc gcgaaca                                                     17


<210>  20
<211>  19
<212>  DNA
<213>  Artificial Sequence

<220>
<223>  synthetic oligonucleotide


<220>
<221>  misc_feature
<222>  (30)..(30)
<223>  not-extensible dideoxy-cytosine

<400>  20
ggtcgagggt ttcgtgggt                                                   19


<210>  21
<211>  229
<212>  DNA
<213>  Artificial Sequence

<220>
<223>  synthetic oligonucleotide

<400>  21
attagagggt ggggtggatt gtgtgtgttt ggtggttgtg gagaggggga gagtaggtag      60

tgggtggtgg ggagtagtat ggagttggtg gtggggagta gtatggagtt tttggttgat     120

tggttggtta tggttgtggt ttggggttgg gtagaggagg tgtgggtgtt gttggaggtg     180

ggggtgttgt ttaatgtatt gaatagttat ggttggaggt tgatttagg                229


<210>  22
<211>  229
<212>  DNA
<213>  Artificial Sequence

<220>
<223>  synthetic oligonucleotide

<400>  22
attagagggt ggggcggatc gcgtgcgttc ggcggttgcg gagaggggga gagtaggtag      60

cgggcggcgg ggagtagtat ggagtcggcg gcggggagta gtatggagtt ttcggttgat     120

tggttggtta cggtcgcggt tcggggtcgg gtagaggagg tgcgggcgtt gttggaggcg     180

ggggcgttgt ttaacgtatc gaatagttac ggtcggaggt cgatttagg                229


<210>  23
<211>  238
<212>  DNA
<213>  Artificial Sequence

<220>
<223>  synthetic oligonucleotide

<400>  23
   tttttttttt tgtgtttgtt tttttttttt tgtagggtta gtgttggggt tttggttgta      60
   gagttatgtg attttggtag gttttgtttg tggggtttgg tgataaggat gtatgatatg     120
   gggtggttag tgtggagttt ggattagtgt agttagatgg gtgggttgag ggttttgtgg     180
   gttttgggga gttgggtgta gagatttttt taagtttgtg tgagttttga gttttttg     238


<210>  24
<211>  238
<212>  DNA
<213>  Artificial Sequence

<220>
<223>  synthetic oligonucleotide

<400>  24
   tttttttttt cgcgttcgtt tttttttttt cgtagggtta gcgttggggt ttcggtcgta      60
   gagttacgtg attttggtag gttttgttcg cggggtttgg cgataaggac gtacgatacg     120
   gggcggttag cgcggagttc ggattagtgt agttagacgg gcgggtcgag ggtttcgtgg     180
   gtttcgggga gtcgggcgta gagatttttt taagtttgtg cgagtttcga gttttttg     238


<210>  25
<211>  21

<212>  DNA
<213>  Artificial Sequence

<220>
<223>  synthetic oligonucleotide

<400>  25
cgctacgaat taccgaatcc c                                                21


<210>  26
<211>  20
<212>  DNA
<213>  Artificial Sequence

<220>
<223>  synthetic oligonucleotide

<400>  26
cgctacctac tctcccctc                                                   20


<210>  27
<211>  18
<212>  DNA
<213>  Artificial Sequence

<220>
<223>  synthetic oligonucleotide

<400>  27
caaaccccgc gaacaaaa                                                    18

## Description

### Field of the invention

The current invention refers to a method for detecting methylation patterns of a nucleotide sequence by an improvement of the LAMP (Loop-mediated Isothermal Amplification) amplification method. As non limitative embodiment the invention refers to the simplex or multiplex detection of methylation patters in the CDKN2A, GATA5 and DAPK1 human genes.

### Background

Hypermethylation of DNA is a common regulation mechanism for gene expression not implying a change of DNA sequence. Specific methyl transferase activities are responsible for the covalent linkage of a methyl-group to cytosines of CpG dinucleotides grouped in CpG rich regions, known as CpG islands¹. CpG islands are usually located within the promoter or the first exon of a gene. Their extensive hypermethylation usually silences the controlled gene by directly impairing the interaction between the DNA molecule and RNA polymerase machinery, including specific transcription factors, or by provoking a local condensation of the chromatin². Aberrant hypermethylation (i.e. silencing) of genes that are responsible for regulation and control of cell cycle, DNA repair, adhesion, angiogenesis, apoptosis, tumor suppressors, may cause abnormal growth and cancer^{3,4,5}. Thus, detection of aberrant methylation patterns of DNA of an individual, either from bodily fluids or from clinical samples, represents a powerful instrument for tumor diagnosis, even at very early stages, prognosis, and therapy planning and control⁶.

The direct detection of DNA methylation can be performed in several ways; for example by methylation sensitive DNA restriction, which exploits the activity of certain methylation sensitive restriction endonucleases which cut only if DNA sequence is free of methylation; limits of this technique are that information is provided only for CpG that are within the restriction cleavage site and moreover risk of partial digestions shall be taken into consideration. Another approach for DNA methylation detection is mass spectrometry that can be used to directly monitor the presence of the methyl-group on DNA bases, but it requires expensive equipments and complex protocols.

Most common methods rely on chemical treatment of DNA by sodium bisulphite. This compound, at suitable conditions, converts cytosines into uracils, unless the cytosine, within a CpG dinucleotide, is methylated; in this case, the base is protected and is not converted⁷. Thus, the method allows the generation of a sequence diversity between originally methylated and unmethylated DNA sequences which can be subsequently exploited for molecular detection. After the bisulphite treatment many techniques are used for a selective detection of a converted or not-converted DNA sequence (originating from unmethylated or methylated DNA molecule).

DNA sequencing following a non selective PCR provides a full analysis of the sample but it is time consuming, not suitable for high throughput screenings and it is not sufficiently sensitive to provide information about samples in which a low amount of methylated DNA is present. In the COBRA analysis a PCR is performed using primers which do not anneal on CpG dinucleotides and the PCR products are then digested by a suitable restriction enzyme which contains CG or TG in its cleavage site⁸. A specific digestion pattern is then obtained, depending on the methylation status of the starting DNA sample (methylated or unmethylated). Disadvantages of this technique are those concerning DNA digestion and multi-step protocols. Methylation specific PCR⁹ allows the specific amplification of either methylated or unmethylated DNA after bisulphite treatment by using primers which anneal on a convenient number of CpG dinucleotides; results are visualized by gel electrophoresis of PCR products which shows the specific amplification band if the expected DNA species was present in the starting DNA sample. This method has an inherent high sensitivity but electrophoresis step makes it contamination-prone and quite time consuming.

Methylight approach¹⁰, consisting in a methylation specific TaqMan real-time PCR, exploits the advantages of real-time PCR assays in terms of sensitivity, quantification capability and high throughput but it still needs an expensive thermo-cycler, a compulsorily fluorescence-based chemistry and at least three hours time reactions.

### Description of the invention

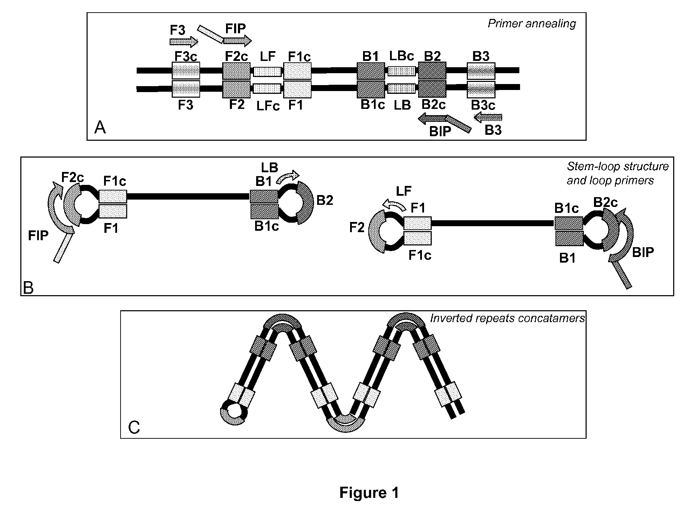

The authors of the current invention have set up a novel method for the detection of methylation patterns that is selective and rapid. The method departs from the LAMP technology, as disclosed in EP 1020534 and WO0177317 herein incorporated by reference and depicted in Fig. 1.

In order to specifically detect hypermethylated DNA, authors set up a novel approach which implements sodium bisulphite treatment and an isothermal method for DNA amplification, the Loop-mediated Isothermal Amplification (LAMP). This technique involves six specific primers and a DNA polymerase with strand displacement activity. LAMP reaction results in a DNA amplification through the formation of intermediate double stem-loop structures and subsequent inverted repeats DNA strands in a continuous synthesis. LAMP offers a well-suited platform for methylation studies as it shows performance similar to PCR but it is much faster than conventional DNA amplification methods (reaction is completed within one hour), it can be easily automated as it needs only a heated reaction block for incubation and it is cheaper and less time consuming as amplification and detection are performed in a single tube. The reaction can be detected in real time by turbidimetry, with no need for fluorescence based chemistries and complex and expensive apparatus for detection. Furthermore a remarkable feature of LAMP method is its extraordinary specificity, due to the eight sequence recognition events required by its peculiar mechanism associated with the isothermal process that prevents exponential amplification of primer-dimers (two of the LAMP primers recognize two regions each).

Therefore LAMP is a convenient method for molecular diagnostics applications, like methylation detection, which require highly specific target recognition.

A statistically significant correlation between epigenetic changes and cancer is defined by the analysis of the aberrant methylation state of more than one gene Thus, a multiplex investigation of the hypermethylation pattern of many genes in a single reaction allows a more complete, more convenient and faster assay. LAMP technology is a highly reliable method for multiplex determinations; in fact primer dimers, which are a major issue in conventional DNA amplifications, are usually not productive in LAMP while the peculiar amplification mechanism reduces the risk of cross reactions on non specific targets. In this way up to three distinct assays, consisting of 18 primers, can be grouped together. Multiplex amplifications are performed and detected either by turbidimetry, for an easy, generic and preliminary methylation screening, or alternatively by fluorescence detection, by using differently labeled oligonucleotides, for the determination of each single amplification event.

Therefore it is an object of the invention a method for detecting patterns of methylation in at least one target nucleic acid sequence comprising a region with at least one CpG dinucleotide, comprising the steps of :
a. obtaining a nucleic acid sample comprising said at least one target nucleic acid sequence;
b. treating said nucleic acid sample with sodium bisulfite in appropriate conditions to obtain the conversion of cytosine residues into uracil residues except for methylated cytosines within a CpG dinucleotide sequence;
c. purifying the sodium bisulphate-treated nucleic acid
d. contacting the purified nucleic acid sample, in appropriate reaction conditions, with a solution comprising a mixture of oligonucleotides and a DNA polymerase with strand displacement activity under isothermal hybridization conditions, wherein said mixture of oligonucleotides consists of primers suitable for a loop amplification mediated polymerization (LAMP) of the target nucleic acid sequence only if the conversion of step b. did not happen, wherein at least one of said primers suitable for LAMP comprises a region that is complementary to the region of the target nucleic acid sequence with at least one CpG dinucleotide, to obtain specific amplified products of the methylated target nucleic acid sequence;
e. detecting of said specific amplified products.

Preferably all of said primers suitable for LAMP comprise a region that is complementary to the region of the target nucleic acid sequence with CpG dinucleotide. More preferably each CpG dinucleotide is not present within three nucleotides from the 3'-end of a F3, B3, F2, B2, LF or LB primer and within three nucleotides from the 5'-end of a F1 or B1 primer.

In a particular embodiment of the invention step e. is achieved by turbidity measurements following precipitation of insoluble pyrophosphate salts, preferably in real-time manner.

In a particular embodiment of the invention step e. is achieved by fluorescence measurements, preferably in real-time manner. The fluorescent signal is generated by at least one fluorescently labelled oligonucleotide contained in the reaction mix; alternatively fluorescent signal is generated by an intercalating dye contained in the solution at item d.; alternatively the fluorescent signal is generated by calcein contained in the solution at item d.

In a particular embodiment of the method of the invention, the target nucleic acid sequence belongs to a gene or to a regulative region thereof linked to a pathologic or pathology-prognostic status. Preferably the gene belongs to the following group: ABCB1, APC, ARHI, BCL2, BRCA1, CALCA, CCND2, CDH1, CDH13, CDKN2A (p16), YP1B1, DAPK1, ESR1, ESR2, GATA1, GATA2, GATA3, GATA4, GATA5, GSTP1, HRAS1, HSD17B4, MCJ, MGMT, MLH1, MYOD1, Pax5a, PGR, PTGS2, RASSF1A, RNR1, SOCS1, SYK, TERT, TFF1, TGFBR2, THBS1, TIMP3, TNFRSF12, TWIST, TYMS, ACTB, ACTB, COL2A1, COL2A1, as depicted in Table 1.

In a preferred embodiment LAMP primers are able of amplifying at least two different target nucleic acid sequences, preferably at least three different target nucleic acid sequences, more preferably said three different target nucleic acid sequences belong to the genes: CDKN2A, DAPK1, GATA5.

In a particular embodiment primers are :
**FZ165** (F3) GGAGGATAGTCGGATCGAGTT (SEQ ID N.3);
**FZ166** (B3) CGCTATCGAAAACCGACCA (SEQ ID N.4);
**FZ176** (FIP) AACGCCGACCCCAAACCCTACGGATTTTGTTTTTTTCGCGGA (SEQ ID N.5);
**FZ177** (BIP) GGGAGGGATTTGCGTTTTTTATTCCAACGCCGAAAACTAACC (SEQ ID N.6):
**FZ178** (LF) CGCTACGAATTACCGAATCCC (SEQ ID N.7);
**FZ179** (LB) AGTTGTGTTTTCGTCGTCGTT (SEQ ID N.8);
**FZ222** (F3) TGGGGCGGATCGCGTG (SEQ ID N.9);
**FZ223** (B3) ACCGTAACTATTCGATACGTTA (SEQ ID N.10);
**FZ224-t** (FIP) CCCGCCGCCGACTCCATACTACTTCGGCGGTTGCGGA (SEQ ID N.11);
**FZ225-t** (BIP) GGAGTTTTCGGTTGATTGGTTGGTCCCCCGCCTCCAACAA (SEQ ID N.12);
**FZ226** (LF) CGCTACCTACTCTCCCCCTC (SEQ ID N.13);
**FZ227** (LB) GGGTCGGGTAGAGGAGGTG (SEQ ID N. 14);
**FZ152** (F3) TCGTAGGGTTAGCGTTGG (SEQ ID N.15);
**FZ147** (B3) CTCGAAACTCGCACAAAC (SEQ ID N.16);
**FZ157-t** (FIP) TAACCGCCCCGTATCGTACGTCGTCGTAGAGTTACGTGATTTTGG (SEQ ID N.17);
**FZ155-t** (BIP) GAGTTCGGATTAGTGTAGTTAGACGGTCTCTACGCCCGACTCC (SEQ ID N.18);
**FZ158** (LF) GCCAAACCCCGCGAACA (SEQ ID N.19);
**FZ151** (LB) GGTCGAGGGTTTCGTGGGT (SEQ ID N.20).

In an alternative particular embodiment primers are :
**FZ165** (F3) GGAGGATAGTCGGATCGAGTT (SEQ ID N.3);
**FZ166** (B3) CGCTATCGAAAACCGACCA (SEQ ID N.4);
**FZ176** (FIP) AACGCCGACCCCAAACCCTACGGATTTTGTTTTTTTCGCGGA (SEQ ID N.5);
**FZ177** (BIP) GGGAGGGATTTGCGTTTTTTATTCCAACGCCGAAAACTAACC (SEQ ID N.6);
**FZ178B** (LF) *BodipyFL*-CGCTACGAATTACCGAATCCC (SEQ ID N.25);
**FZ179** (LB) AGTTGTGTTTTCGTCGTCGTT (SEQ ID N.8);
**FZ222** (F3) TGGGGCGGATCGCGTG (SEQ ID N.9);
**FZ223** (B3) ACCGTAACTATTCGATACGTTA (SEQ ID N.10);
**FZ224-t** (FIP) CCCGCCGCCGACTCCATACTACTTCGGCGGTTGCGGA (SEQ ID N.11);
**FZ225-t** (BIP) GGAGTTTTCGGTTGATTGGTTGGTCCCCCGCCTCCAACAA (SEQ ID N.12);
**FZ226T** (LF) *TAMRA*-CGCTACCTACTCTCCCCCTC (SEQ ID N.26);
**FZ227** (LB) GGGTCGGGTAGAGGAGGTG (SEQ ID N. 14);
**FZ152** (F3) TCGTAGGGTTAGCGTTGG (SEQ ID N.15);
**FZ147** (B3) CTCGAAACTCGCACAAAC (SEQ ID N.16);
**FZ157-t** (FIP) TAACCGCCCCGTATCGTACGTCGTCGTAGAGTTACGTGATTTTGG (SEQ ID N.17);
**FZ155-t** (BIP) GAGTTCGGATTAGTGTAGTTAGACGGTCTCTACGCCCGACTCC (SEQ ID N.18);
**FZ291B650** (LF) *Bodipy650*/*665-*CAAACCCCGCGAACAAAA (SEQ ID N.27);
**FZ151** (LB) GGTCGAGGGTTTCGTGGGT (SEQ ID N.20).

The Methylation Specific Loop-mediated Amplification refers to a method for specific, sensitive and selective detection and amplification of hypermethylated DNA in any region of interest, in this specific case in the promoter regions of human genes CDKN2A, GATA5 and DAPK1, in simplex and triplex formats, comprising the steps of
a. Obtaining a nucleic acid sample from a human patient.
b. Treating said sample with sodium bisulphite in the appropriate conditions defined by the protocol of the utilized commercial kit.
c. Purifying the said modified DNA.
d. Contacting said nucleic acid with a solution comprising a mixture of oligonucleotides and a DNA polymerase with strand displacement activity under hybridization conditions, wherein
   I. The said mixture of oligonucleotides includes primers suitable for a loop mediated isothermal amplification of a region of the promoter or of the first exon of a gene of interest. This oligonucleotide mixture includes two outer primers F3 and B3 and two inner primers FIP and BIP where said FIP includes two oligonucleotide sequences (F2 and F1c) designed to recognize two different regions of the target sequence and said BIP includes two oligonucleotide sequences (B2 and B1c) designed to recognize two different regions of the target sequence. Moreover the oligonucleotides mixture contains two loop primers, LF and LB which are designed to recognize target regions comprised respectively between F2 and F1 and B2 and B1.
   II. Said LAMP primer sets for hypermethylation detection are designed to be specific only for the methylated version of the DNA sequence after the bisulfite treatment; primers anneal on their target in regions comprising a convenient number of CpG dinucleotides which confer to the assay the capability to discriminate between methylated and unmethylated DNA sequence. In this way LAMP primers can anneal and extend only when their specific target is encountered, while the mismatches which occur on the aspecific target prevent annealing and extension. Due to the simultaneous probing by 8 priming regions on a high number of CpG dinucleotides, a high specificity is assured by the technique.
   III. The said LAMP primer set for hypermethylation detection where LAMP primers do not contain CpG dinucleotides within 3 nucleotides from the 3'-end of the F3, B3, F2, B2, LF, LB primer regions and within 3 nucleotides from the 5'-end of the F1 and B1 primer regions. By keeping the position of the CpG dinucleotides distant from the 3'-end of the primers, the effect of a single mismatch on priming efficiency is reduced. In this way, mismatches between the primer and one or more positions on the target do not abolish completely the priming efficiency but only reduce it. Thereby partial methylation, which is frequently encountered in cancerous tissues and which can impair DNA amplification in a regular PCR approach, can still lead to a detectable LAMP product, albeit having smaller yield than a fully methylated target that fits the primer exactly. At the same time, target specificity (i.e. absence of mispriming at non-target regions) is maintained because LAMP operates only if all 6 primers bind to the target. Accordingly, by using 6 primers, LAMP achieves both target specificity and quantitative amplification of partly methylated target regions.
   IV. Said LAMP primers set is the only one constituting the mixture or alternatively said mixture is constituted by two or more primer sets which are specific for different regions of human genome and this "multiplex" reaction amplifies more than one genomic region at the same time.
   V. Said DNA polymerase is Bst large fragment polymerase or a combination of: Bca (exo-), Vent, Vent (exo-), Deep Vent, Deep Vent (exo-), φ29 phage, MS-2 phage, Z-Taq, KOD, Klenow fragment.
e. Incubating the resulting mixture at a constant temperature comprised between 50 and 70°C.
f. Detecting a signal indicative of the amplification of the DNA target sequence by the specific primer set(s), wherein detection of said signal can be achieved by:
   I. Observing the increase of turbidity of the reaction tube, due to the remarkable precipitation of magnesium pyrophosphate deriving from the incorporation of the dNTPs in the synthesized strands either
      i. At end-point by a single turbidity measure or simply by visual inspection of the reaction tube
      ii. In a real-time fashion by performing the amplification in a real-time turbidimeter, consisting in a thermo-block that allows continuous monitoring of transmittance.
   II. Detecting the fluorescence emitted by the reaction mixture either at end point or in real time, by measuring
      i. the increasing fluorescence emission of an intercalating dye as it is progressively incorporated in the amplified growing strands
      ii. the increasing fluorescence emission of peculiar dyes such as calcein which emits a fluorescent signal as the weakly bound manganese ion is progressively sequestered by the pyrophosphate ion and precipitates as an insoluble salt.
      iii. the quenching of the fluorescence emitted by one or more fluorescently labelled oligonucleotides specific for a region of LAMP amplification products. These oligonucleotides emit fluorescence when they are not bound to the target sequence; after the annealing of these labeled oligonucleotides on their target sequence and eventually their elongation by the polymerase and incorporation into the final amplification product, the fluorescence emission is progressively quenched, especially when the fluorophore is positioned in the proximity of one or more guanine nucleotides. The time needed for the fluorescence to be quenched down to a fixed threshold level is inversely correlated to initial target amount. More than one differently labelled fluorescent oligonucleotides, specific for different target genes can be used together with corresponding primers sets (eventually substituting one of the regular loop primers) at the same time. In this way a multiplex reaction is obtained, where the individual events of amplification can be distinguished by measuring the emitted fluorescent signal of each marked oligonucleotide at the appropriate emission wavelength.

Table 1 refers to exemplificative genes that contain target sequences suitable to be amplified with the method of the invention.

**Table 1**

| abbreviation | alias | full name | Gene ID | HGNC ID | RefSeq ID | Acc. no. |
|---|---|---|---|---|---|---|
| ABCB1 | | ATP-binding cassette, sub-family B (MDR/TAP), member 1 | 5243 | 40 | NM_000927 | M14758 |
| ACTB | | actin, beta | 60 | 132 | NM_001101 | M28424 |
| APC | | adenomatous polyposis coli | 324 | 583 | NM_000038 | M74088 |
| DIRAS3 | ARHI | DIRAS family, GTP-binding RAS-like 3 | 9077 | 687 | NM_004675 | U96750 |
| BCL2 | | B-cell CLL/lymphoma 2 | 596 | 990 | NM_000657 | M14745 |
| BRCA1 | | breast cancer 1, early onset | 672 | 1100 | | U14680 |
| CALCA | | calcitonin-related polypeptide alpha | 796 | 1437 | NM_001741 | X00356 |
| CCND2 | | cyclin D2 | 894 | 1583 | NM_001759 | AF518005 |
| CDH1 | | cadherin 1, type 1, E-cadherin (epithelial) | 999 | 1748 | NM_004360 | L08599 |
| CDH13 | | cadherin 13, H-cadherin (heart) | 1012 | | 1753NM_001257 | U59288 |
| CDKN2A | | cyclin-dependent kinase inhibitor 2A (melanoma, p16, inhibits CDK4) | 1029 | 1787 | NM_000077 | L27211 |
| COL2A1 | | collagen, type II, alpha 1 | 1280 | 2200 | NM_001844 | X16468 |
| DAPK1 | | death-associated protein kinase 1 | 1612 | 2674 | NM_004938 | X76104 |
| ESR1 | | estrogen receptor 1 | 2099 | 3467 | | X03635 |
| ESR2 | | estrogen receptor 2 (ER beta) | 2100 | 3468 | | X99101 |
| GATA1 | | GATA binding protein 1 (globin transcription factor 1) | 2623 | 4170 | NM_002049 | X17254 |
| GATA2 | | GATA binding protein 2 | 2624 | 4171 | NM_032638 | AF169253 |
| GATA3 | | GATA binding protein 3 | 2625 | 4172 | M_001002295 | X55122 |
| GATA4 | | GATA binding protein 4 | 2626 | 4173 | NM_002052 | AK097060 |
| GATA5 | | GATA binding protein 5 | 8 | 14062 15802 | NM_080473 | BC117356 |
| GSTP1 | | glutathione S-transferase pi 1 | 2950 | 4638 | | U12472 |
| HRAS1 | | v-Ha-ras Harvey rat sarcoma viral oncogene homolog | 3265 | 5173 | NM_176795 | AJ437024 |
| HSD17B4 | | hydroxysteroid (17-beta) dehydrogenase 4 | 3295 | 5213 | NM_000414 | |
| DNAJC15 | MCJ | DnaJ (Hsp40) homolog, subfamily C, member 15 | 29103 | 20325 | M_013238 | AF126743 |
| MGMT | | O-6-methylguanine-DNA methyltransferase | 4255 | 7059 | NM_002412 | M29971 |
| MLH1 | | mutL homolog 1, colon cancer, nonpolyposis type 2 (E. coli) | 4292 | 7127 | NM_000249 | U07418 |
| MYOD1 | | myogenic differentiation 1 | 4654 | 7611 | NM_002478 | AF027148 |
| Pax5a | | paired box 5 | 5079 | 8619 | NM_016734 | |
| PGR | | progesterone receptor | 5241 | 8910 | NM_000926 | M15716 |
| PTGS2 | | prostaglandin-endoperoxide synthase 2 (prostaglandin G/H synthase and cyclooxygenase) | 5743 | 9605 | NM_000963 | D28235 |
| RASSF1A | | Ras association (RalGDS/AF-6) domain family member 1 | 11186 | 9882 | NM_170714 | AF132675 |
| RNR1 | | RNA, ribosomal 1 | 6052 | 10082 | | X01547 |
| SOCS1 | | suppressor of cytokine signaling 1 | 8651 | 19383 | NM_003745 | U88326 |
| SYK | | spleen tyrosine kinase | 6850 | 11491 | NM_001135052 | L28824 |
| TERT | | telomerase reverse transcriptase | 7015 | 11730 | NM_198253 | AF015950 |
| TFF1 | | trefoil factor 1 | 7031 | 11755 | NM_003225 | BC032811 |
| TGFBR2 | | transforming growth factor, beta receptor II (70/80kDa) | 7048 | 11773 | NM_003242 | |
| THBS1 | | thrombospondin 1 | 7057 | 11785 | NM_003246 | |
| TIMP3 | | TIMP metallopeptidase inhibitor 3 | 7078 | 11822 | NM_000362 | |
| TNFRSF12A | | tumor necrosis factor receptor superfamily, member 12A | 51330 | 18152 | M_016639 | AB035480 |
| TWIST1 | | twist homolog 1 (Drosophila) | 7291 | 12428 | NM_000474 | U80998 |
| TYMS | | thymidylate synthetase | 7298 | 12441 | NM_001071 | X02308 |
| CYP1B1 | | cytochrome P450, family 1, subfamily B, polypeptide 1 | 1545 | 2597 | NM_000104 | U56438 |

The invention will be described with reference to specific not limiting examples, including the following figures:
**Figure 1****. LAMP principle** The basic reaction is performed by 4 primers specific for 6 regions of a target genomic sequence. Internal primers anneal and extend on the target; the product is displaced in two steps by external primers (F3, B3) and forms a double stem-loop structure (*starting structure*) (panel A). The starting structure is simultaneously amplified from its free 3' and by another internal primer (panel B). DNA concatamers consisting of inverted repeats of the initial module are progressively synthesized in an exponential fashion (panel C). Additional loop primers anneal in the indicated regions (panel A, B) and increase the yield of amplification, resulting in a faster and more efficient reaction.

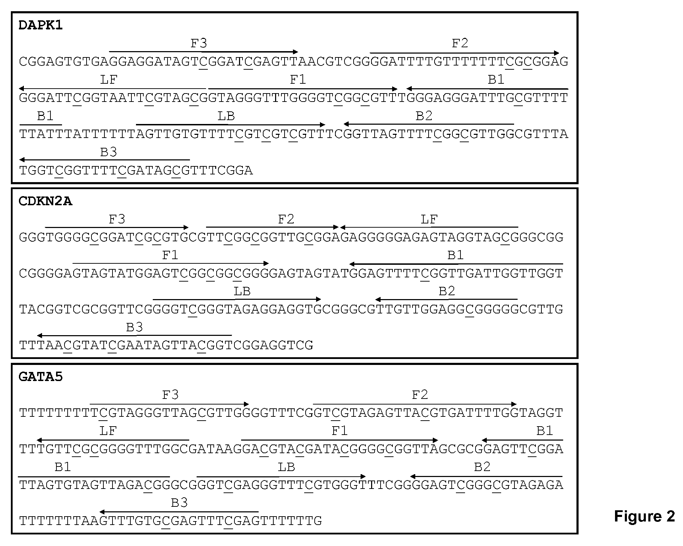

**Figure 2****. Scheme of primers location in CDKN2A, DAPK1 and GATA5 promoter regions** Primers sets specific for promoter regions of human CDKN2A (NCBI GeneID: 1029), DAPK1 (NCBI GeneID: 1612) and GATA5 (NCBI GeneID: 140628) genes were designed as depicted. Primers were designed to be specific only for the methylated version of each promoter. Among all possible primer sets those containing a convenient number of CpG dinucleotides were selected, in order to discriminate as much as possible between methylated and unmethylated sequences. Moreover no CpG was included within 3 nucleotides from the 3'-end of the F3, B3, F2, B2, LF, LB primer regions and within 3 nucleotides from the 5'-end of the F1 and B1 primer regions, in order to moderate the effect of a mismatch on priming efficiency in case of partial methylation.

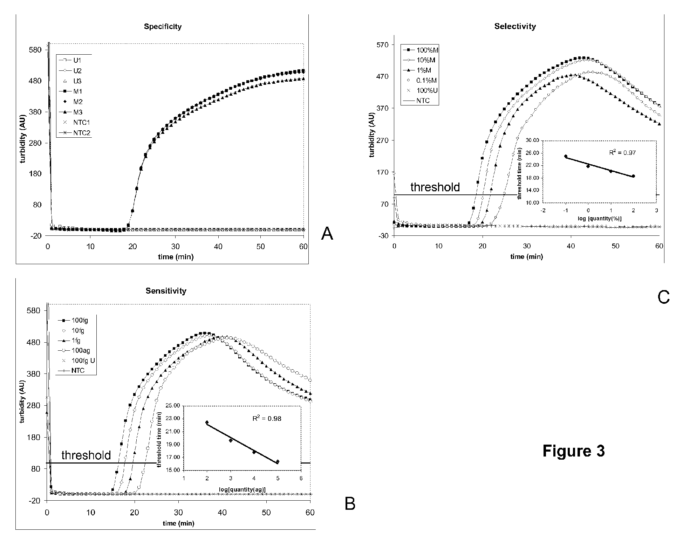

**Figure 3****. Proof of principle by DAPK1 assay on plasmid target. Specificity, sensitivity and selectivity** To evaluate reliability, specificity and performance of MS-LAMP for hypermethylation detection without the variability introduced by the bisulphite treatment, MS-LAMP DAPK1 assay was first tested on two synthetic plasmids incorporating the expected sequence of DAPK1 promoter following bisulphite treatment either in the fully methylated and unmethylated versions. For specificity assay (panel A), 15000 copies/reaction (cps/rxn) of unmethylated and methylated plasmid were included as reaction target, along with no target controls. For sensitivity assay (panel B) MS-LAMP DAPK1 reaction was conducted on 10-fold serial dilutions of methylated plasmid between 100 fg/rxn (approx. 3*10⁴ cps/rxn) and 10 ag/rxn (approx. 3 cps/rxn). In the inset of panel B the threshold time for (reaction time when amplification curve crosses a fixed turbidity threshold=100AU) is plotted versus the logarithm of target amount for each dilution. For selectivity assay (panel C) MS-LAMP DAPK1 reaction was conducted on dilutions of methylated plasmid (M) in an unmethylated plasmid (U) background, where the M plasmid represented from 100% to 0.01 % of the total DNA. Total DNA amount in each target mix was 100 fg/rxn. All the amplification curves are depicted as turbidity signal versus reaction time. In the inset of panel C the threshold time is plotted versus the logarithm of the percentage of M target contained in each dilution.

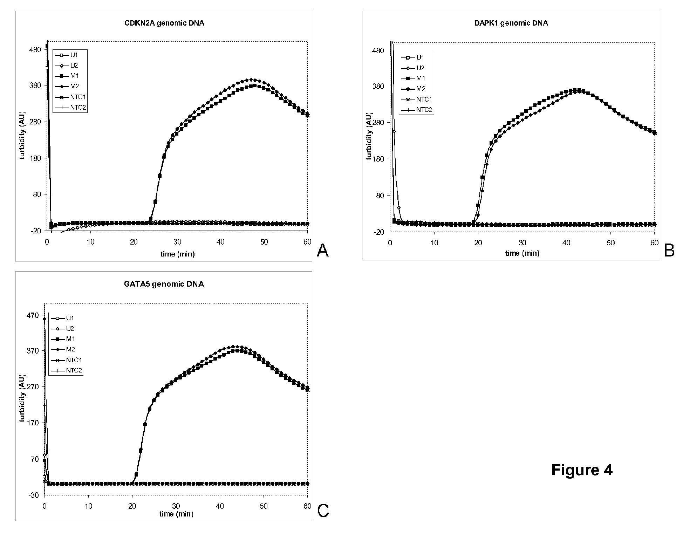

**Figure 4****. Specificity of CDKN2A, DAPK1 and GATA5 assays on human genomic DNA**. Specificity of MS-LAMP for the promoters of CDKN2A (panel A), DAPK1 (panel B) and GATA5 (panel C) was evaluated using commercial human genomic DNA as a target either in fully methylated and unmethylated form. Prior to testing, both samples were treated with sodium bisulphite to convert the non-methylated cytosines into uracils and 2 µl of treated DNA, corresponding approximately to 50 ng, were added to LAMP reactions. Results of amplification reactions, based on three or more independent experiments, are shown as turbidity signal versus reaction time.

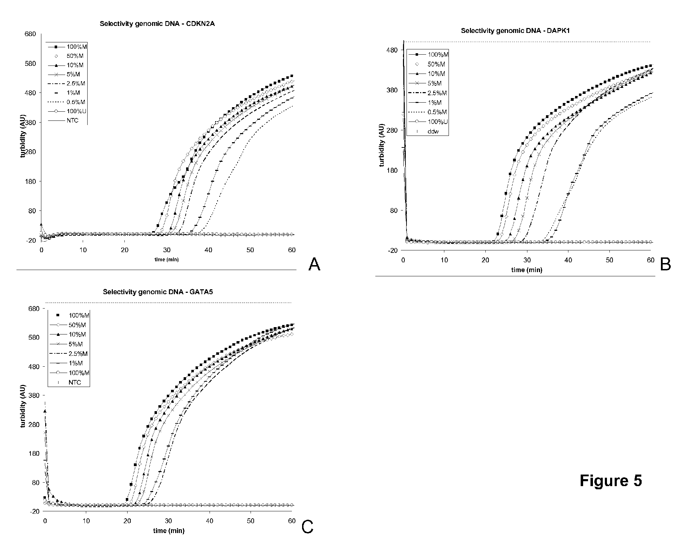

**Figure 5****. Selectivity of CDKN2A, DAPK1 and GATA5 assays on human genomic DNA**. Selectivity of MS-LAMP for the promoters of CDKN2A (panel A), DAPK1 (panel B) and GATA5 (panel C) was evaluated on progressive dilutions of fully methylated DNA in a background of unmethylated DNA, resulting in mixtures containing from 100% to 0.25% of methylated DNA. The obtained mixtures were then treated by sodium bisulphite and 50 total ng of each preparation were added the reactions. Turbidimetry amplification curves for each M/U ratio down to the lowest reproducible (3 repetitions) M/U ratio are shown.

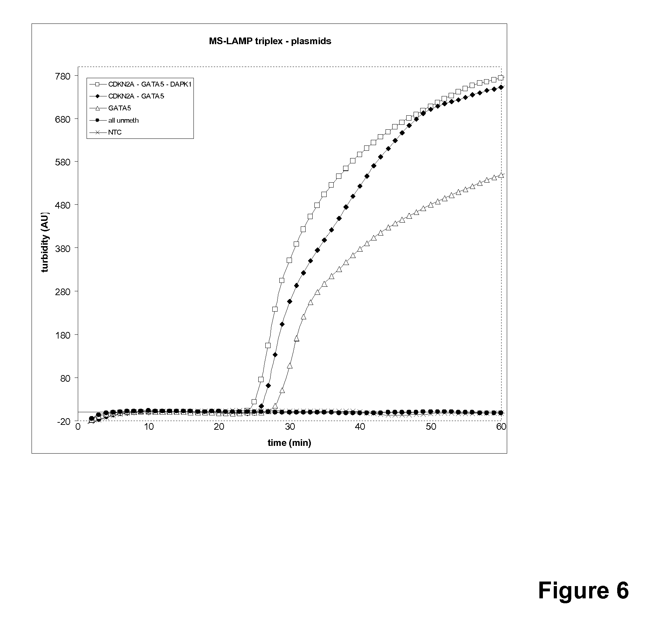

**Figure 6****. Multiplex MS-LAMP**. Triplex MS-LAMP, composed by the three primer sets (18 primers) CDKN2A-GATA5-DAPK1 gathered in a single reaction, was evaluated in turbidimetry in presence of plasmid controls appropriately mixed as follows: all three unmethylated targets, one methylated (GATA5) and two unmethylated (CDKN2A and DAPK1) targets, two methylated (GATA5 and CDKN2A) and one unmethylated (DAPK1) target, all the three methylated targets. A no target control was also included. Reactions contained 15000 cps/rxn of each plasmid.

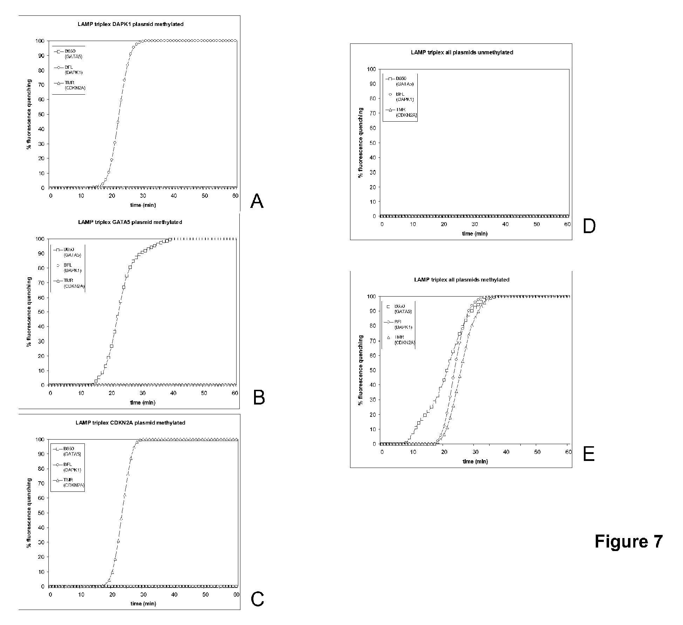

**Figure 7****. Multiplex MS-LAMP and detection by fluorescence**. Triplex MS-LAMP was implemented with fluorescently labelled primers, each one substituting one of the two loop primers of each set of primers. Different fluorescent dyes (BodipyFL, Bodipy650/665 and TAMRA) were used for the three assays (respectively DAPK1, GATA5 and CDKN2A) and the triplex reaction was run under isothermal conditions in a real-time thermocycler. The fluorescence emitted by each dye is progressively quenched as the the primer which it is linked to, is progressively incorporated into the final amplification product. Capability of the triplex reaction to amplify each single methylated target was evaluated on different plasmids mixtures containing separately each methylated plasmid in presence of the unmethylated form of the remaining two plasmids (panels A, B, C), and all the three unmethylated (panel D) or methylated (panel E) plasmids at the same time. Results are shown as % of fluorescence quenching versus reaction time.

### Example 1 Materials, methods and results of the proof of principle by DAPK1 assay on plasmidic target. Specificity, sensitivity and selectivity

### Reagents

### DAPK1 plasmids controls (synthesized by GeneArt);

Unmethylated plasmid (U), containing part of the expected sequence of the completely unmethylated human DAPK1 gene promoter after complete bisulfite conversion.
Insert sequence (SEQ ID N.1): 
5’TGGAGTGTGAGGAGGATAGTTGGATTGAGTTAATGTTGGGGATTTTGTTTTTTTT
GTGGAGGGGATTTGGTAATTTGTAGTGGTAGGGTTTGGGGTTGGTGTTTGGGAGG
GATTTGTGTTTTTTATTTATTTTTTAGTTGTGTTTTTGTTGTTGTTTTGGTTAGTTTTT
GGTGTTGGTGTTTATGGTTGGTTTTTGATAGTGTTTTGGAGGG3’
 Methylated plasmid (M), containing part of the expected sequence of the fully methylated human DAPK1 gene promoter after complete bisulfite conversion.
Insert sequence (SEQ ID N.2): 
5'CGGAGTGTGAGGAGGATAGTCGGATCGAGTTAACGTCGGGGATTTTGTTTTTTC
GCGGAGGGGATTCGGTAATTCGTAGCGGTAGGGTTTGGGGTCGGCGTTTGGGAG
GGATTTGCGTTTTTTATTTATTTTTTAGTTGTGTTTTCGTCGTCGTTTCGGTTAGTTT
TCGGCGTTGGCGTTTATGGTCGGTTTTCGATAGCGTTTCGGAGGG3'
 Primers: synthesized by Eurofins MWG Operon, referred to as *"primers":*
**FZ165** (F3) GGAGGATAGTCGGATCGAGTT (SEQ ID N.3)
**FZ166** (B3) CGCTATCGAAAACCGACCA (SEQ ID N.4)
**FZ176** (FIP) AACGCCGACCCCAAACCCTACGGATTTTGTTTTTTTCGCGGA (SEQ ID N.5)
**FZ177** (BIP) GGGAGGGATTTGCGTTTTTTATTCCAACGCCGAAAACTAACC (SEQ ID N.6)
**FZ178** (LF) CGCTACGAATTACCGAATCCC (SEQ ID N.7)
**FZ179** (LB) AGTTGTGTTTTCGTCGTCGTT (SEQ ID N.8)
Reaction buffer: 200mM Tris HCl pH 8.8, 100mM KCl, 80mM MgSO₄, 100mM (NH₄)2SO₄, 1 % Tween, *"buffer 10x"* dNTPs mix 25mM (Fermentas), *"dNTPs"*
Betaine 5M (Sigma Aldrich), *"betaine"*
Bst Large Fragment polymerase 8U/ul (New England Biolabs), *"Polymerase"*
Sterile apyrogen water (SALF Spa), *"ddw"*

### Material

Turbidimeter (Teramecs LA-200) for incubation at constant temperature and real time monitoring of turbidity

### Procedure

### 1. Sample preparation

Stock the primers in aliquots. It is better to store stock solutions at -20°C, while working dilutions should be stored at 4°C

### - Specificity

Prepare reaction mix as follows: 0.2µM outer primers (F3 and B3), 1.6µM inner primers (FIP and BIP), 0.8uM loop primers (LF and LB), 1x buffer solution, 1.4mM dNTPs mix, 0.8M betaine, 8 U Bst Polymerase. Final volume of the reaction mix must be 25 ul, (24 ul reaction mix and 1 ul sample). Always keep reagents on ice. Prepare the mix for at least 8 samples, comprising three U plasmid samples, three M plasmid samples (15000 cps plasmid/rxn) and two no target control.

Dispense 24µl of reaction mix in the reaction tube. Keep the reaction tubes on ice. Always keep the reaction mix on ice from now on. Change gloves.

Prepare plasmids solution at the appropriate concentration (15000 cps/ul) from shipped solution (wt plasmid and mut plasmid). Shipped solution is a 4*10¹⁰ copies/µl

### - Sensitivity

Prepare reaction mix as follows: 0.2µM outer primers (F3 and B3), 1.6µM inner primers (FIP and BIP), 0.8uM loop primers (LF and LB), 1x buffer solution, 1.4mM dNTPs mix, 0.8M betaine, 8 U Bst Polymerase. Final volume of the reaction mix must be 25 ul, (24 ul reaction mix and 1 ul sample). Always keep reagents on ice. Prepare the mix for at least 20 samples, comprising three 100 fg/rxn M plasmid samples, three 10 fg/rxn M plasmid samples, three 1 fg/rxn M plasmid samples, three 100 ag/rxn M plasmid samples, three 10 ag/rxn M plasmid samples, three 100 fg/rxn U plasmid samples and two no target control.

Dispense 24µl of reaction mix in the reaction tube. Keep the reaction tubes on ice. Always keep the reaction mix on ice from now on. Change gloves.

Prepare dilutions in Tris-EDTA-yeastRNA buffer (Tris 10 mM, EDTA 1 mM, yeastRNA 20 ng/ul) of the U and M plasmidic targets (*"target dilutions*") from shipped solutions. Shipped solution is approximately 4*10¹⁰ copies/µl. Dilute U plasmid to 100 fg/µl (approximately 3*10⁴ CPS/µl). Dilute M plasmid to 100 fg/µl (approximately 3*10⁴ CPS/µl) and then by 10 fold serial dilutions to 10 ag/rxn (approximately 3 cps/pl). Pre denature each target preparation for 10 minutes at 95°C and then immediately chill on ice for at least 10 minutes.

Add 1µl of target dilutions to the reaction tubes, in triplicate. Add 1ul of water in the no target controls samples, in duplicate. Close all the tubes.

### - Selectivity

Prepare reaction mix as follows: 0.2µM outer primers (F3 and B3), 1.6µM inner primers (FIP and BIP), 0.8uM loop primers (LF and LB), 1x buffer solution, 1.4mM dNTPs mix, 0.8M betaine, 8 U Bst Polymerase. Final volume of the reaction mix must be 25 ul, (24 ul reaction mix and 1 ul sample). Always keep reagents on ice. Prepare the mix for at least 20 samples, comprising three 100% M plasmid samples, three 10% M plasmid samples, three 1% M plasmid samples, three 0.1% M plasmid samples, three 0.01% M plasmid samples, three 100% U plasmid samples and two no target control.

Dispense 24µl of reaction mix in the reaction tube. Keep the reaction tubes on ice. Always keep the reaction mix on ice from now on. Change gloves.

Prepare dilutions in Tris-EDTA-yeastRNA buffer (Tris 10 mM, EDTA 1 mM, yeastRNA 20 ng/ul) of the U and M plasmidic targets ("*target dilutions*") from shipped solutions. Shipped solution is approximately 4*10¹⁰ copies/µl. Dilute U plasmid to 100 fg/µl (approximately 3*10⁴ cps/µl). Dilute M plasmid to 100 fg/µl (100%M) and then serially dilute each time in U plasmid at 100 fg/µl by 4 10-fold serial dilutions to 0.01 %M. Pre denature each target preparation for 10 minutes at 95°C and then immediately chill on ice for at least 10 minutes.

Add 1µl of target dilutions to the reaction tubes, in triplicate. Add 1ul of water in the no target controls samples, in duplicate. Close all the tubes.

### 2. Reaction

Program the turbidimeter in order to obtain a constant reaction temperature of 63°C for 1 hour. Put the reaction tubes in the instrument immediately before the beginning of the programs. Start the program.

### 3. Data analysis

Analyze the raw absorbance data using a customized MS Excel macro for baseline correction and threshold time calculation.

### Results

In order to evaluate reliability, specificity and performance of MS-LAMP for hypermethylation detection without the variability introduced by the bisulphite treatment, we ordered two synthetic plasmids incorporating the expected sequence of DAPK1 promoter following bisulphite treatment either in the fully methylated and unmethylated versions (hereinafter named methylated and unmethylated plasmids, for ease). Plasmids were assayed in real time turbidimetry reactions, where turbidity of each reaction is measured every 6 seconds and can be therefore plotted versus reaction time in a real-time graph. Specificity of LAMP primer set for methylated DAPK1 target was investigated including 15000 cps/rxn of unmethylated and methylated plasmid along with no target controls: amplification occurred exclusively starting from the plasmid representing methylated DAPK1 promoter while plasmid representing unmethylated sequence and no-target-control (NTC) produced no amplification within one hour reaction (figure 3A).

These results indicate that MS-LAMP can discriminate sequences based on their original difference in methylation status of CpG islands.

To evaluate the sensitivity of MS-LAMP DAPK1, the assay was conducted on 10-fold serial dilutions of methylated plasmid in water between 100 fg/rxn (approx. 3*10⁴ cps/rxn) and 10 ag/rxn (approx. 3 cps/rxn). Representative curves for each set of repetitions are depicted down to the lowest dose that was always detected. Amplification was reproducibly obtained down to 100 ag/rxn with good linearity as semilogarithmic graph shows, where threshold time of each reaction is plotted against log of target amount (R²= 0.98) (figure 3B).

In order to evaluate the DAPK1 assay selectivity, that is the lowest amount of specific target detectable in an aspecific background, the assay was conducted on dilutions of methylated plasmid (M) in an unmethylated plasmid (U) background, ranging from 100%M to 0.01 %M in U plasmid. Total DNA amount in each target mix was 100 fg/rxn. Representative curves for each set of repetitions are depicted down to the lowest dose that was always detected. LAMP was able to reproducibly detect down to 0.1% M plasmid diluted in U plasmid in a quantitative fashion (R²=0.97) (figure 3C).

### Example 2 Materials, methods and results of specificity of CDKN2A, DAPK1 and GATA5 assays on human genomic DNA

### Reagents

CpGenome Universal Methylated (referred as M) and Unmethylated (referred as U) human genomic DNA (Millipore)
Bisulphite treatment kit: EZ-DNA Methylation Direct kit (Zymo Research)
Primers: synthesized by Eurofins MWG Operon, referred to as *"primers"*:
DAPK1 PRIMER SET
As in **Example 1.**
CDKN2A PRIMER SET
**FZ222** (F3) TGGGGCGGATCGCGTG (SEQ ID N.9)
**FZ223** (B3) ACCGTAACTATTCGATACGTTA (SEQ ID N.10)
**FZ224-t** (FIP) CCCGCCGCCGACTCCATACTACTTCGGCGGTTGCGGA (SEQ ID N.11)
**FZ225-t** (BIP) GGAGTTTTCGGTTGATTGGTTGGTCCCCCGCCTCCAACAA (SEQ ID N.12)
**FZ226** (LF) CGCTACCTACTCTCCCCCTC (SEQ ID N.13)
**FZ227** (LB) GGGTCGGGTAGAGGAGGTG (SEQ ID N. 14) GATA5 PRIMER SET
**FZ152** (F3) TCGTAGGGTTAGCGTTGG (SEQ ID N.15)
**FZ147** (B3) CTCGAAACTCGCACAAAC (SEQ ID N.16)
**FZ157-t** (FIP) TAACCGCCCCGTATCGTACGTCGTCGTAGAGTTACGTGATTTTGG (SEQ ID N.17)
**FZ155-t** (BIP) GAGTTCGGATTAGTGTAGTTAGACGGTCTCTACGCCCGACTCC (SEQ ID N.18)
**FZ158** (LF) GCCAAACCCCGCGAACA (SEQ ID N.19)
**FZ151** (LB) GGTCGAGGGTTTCGTGGGT (SEQ ID N.20)
Reaction buffer: 200mM Tris HCl pH 8.8, 100mM KCl, 80mM MgSO4, 100mM (NH4)2SO4, 1% Tween, *"buffer 10x"*
dNTPs mix 25mM (Fermentas), *"dNTPs"*
Betaine 5M (Sigma Aldrich), *"betaine"*
Bst Large Fragment polymerase 8U/µl (New England Biolabs), *"Polymerase"*
Sterile apyrogen water (SALF Spa), *"ddw"*

### Material

Turbidimeter (Teramecs LA-200) for incubation at constant temperature and real time monitoring of turbidity
Thermal block for incubation of bisulphite conversion reaction

### Procedure

### Sample preparation

### Reaction

### Data Analysis

### 1. sample preparation

Treat 500 ng of both fully methylated and fully unmethylated human genomic DNA (100 ng/µl) by EZ-DNA Methylation Direct kit following the manufacturer'sr protocol. Elute the DNA from purification columns using 20 µl of Tris-EDTA buffer (10 mM Tris, 1 mM EDTA). Final theoretical concentration is 25 ng/µl.

Stock the primers in aliquots. It is better to store stock solutions at -20°C, while working dilutions should be stored at 4°C

Prepare three independent reaction mixes for the three assays as follows: 0.2µM outer primers (F3 and B3), 1.6µM inner primers (FIP and BIP), 0.8uM loop primers (LF and LB), 1x buffer solution, 1.4mM dNTPs mix, 0.8M betaine, 8 U Bst Polymerase. Final volume of the reaction mix must be 25 ul, (23 ul reaction mix and 2 ul sample). Always keep reagents on ice. Prepare the mix for at least six samples, comprising two unmethylated human genomic DNA samples, two methylated human genomic DNA samples and two no target controls for each assay.

Add 2µl (50 ng) of treated human genomic DNA to the reaction tubes, in duplicate for both fully methylated and unmethylated human genomic DNA. Add 2ul of water in the no target controls samples, in duplicate. Close all the tubes.

### 2. Reaction

Program separately the three blocks of the turbidimeter in order to obtain a constant reaction temperature of 63°C for DAPK1 and GATA5 assays and of 65°C for CDKN2A assay for 1 hour.

Put the reaction tubes in the instrument immediately before the beginning of the programs. Start the program.

### 3. Data analysis

Analyze the raw absorbance data using a customized MS Excel macro for baseline correction and threshold time calculation.

### Results

The three assays were evaluated for their capability to discriminate between hypermethylated and unmethylated target after a bisulphite treatment. The target used in these experiments is a commercial human genomic DNA which is purchased in fully methylated form (enzymatically treated by the manufacturer in order to have all the CpGs of the genome in their methylated form) and completely unmethylated form (with no methylation in any of the CpGs). Genomic DNA were treated by us using a commercial bisulfite treatment kit, following the suggested protocol, in order to generate the sequence differences between the two forms and to allow the discrimination by our methylation specific LAMP primer sets between methylated and unmethylated DNA. 50 ng of each DNA type were assayed by each primer set in duplicate in at least 3 independent experiments, and amplification curves obtained by real time turbidimetry showed a perfect discrimination between the two genomic forms after the bisulphite treatment. Only the intended (methylated) genomic DNA was amplified by CDKN2A (figure 4A), DAPK1 (figure 4B) and GATA5 (figure 4C) primer sets within one hour reaction.

### Example 3 Materials, methods and results of selectivity of CDKN2A, DAPK1 and GATA5 assays on human genomic DNA

### Reagents

CpGenome Universal Methylated (referred as M) and Unmethylated (referred as U) human genomic DNA (Millipore)
Bisulphite treatment kit: EZ-DNA Methylation Direct kit (Zymo Research)
Primers: synthesized by Eurofins MWG Operon, referred to as *"primers":*
DAPK1 PRIMER SET
As in **Example 1.**
CDKN2A PRIMER SET
As in **Example 2.**
GATA5 PRIMER SET
As in **Example 2.**
Reaction buffer: 200mM Tris HCl pH 8.8, 100mM KCl, 80mM MgSO₄, 100mM (NH₄)2SO₄, 1 % Tween, *"buffer 10x"*
dNTPs mix 25mM (Fermentas), *"dNTPs"*
Betaine 5M (Sigma Aldrich), *"betaine"*
Bst Large Fragment polymerase 8U/ul (New England Biolabs), *"Polymerase"*
Sterile apyrogen water (SALF Spa), *"ddw"*

### Material

Turbidimeter (Teramecs LA-200) for incubation at constant temperature and real time monitoring of turbidity
Thermal block for incubation of bisulphite conversion reaction

### Procedure

### Sample preparation

### Reaction

### Data Analysis

### 1. sample preparation

Starting from 100 ng/µl stocks of fully methylated and unmethylated commercial genomic DNA (respectively 100%M and 100%U) appropriately dilute fully methylated genomic DNA in a background of completely unmethylated DNA, in order to obtain 100 ng/µl DNA mixtures containing different ratios of methylated DNA: 50%M, 10%M, 5%M, 2.5%M, 1%M, 0.5%M 0.25%M .

Treat 500 ng (5 µl) of all the obtained mixes along with 100%M and 100%U genomic DNA as controls with EZ-DNA Methylation Direct kit following the manufacturer protocol. Finally elute the DNA from purification columns using 20 µl of Tris-EDTA buffer (10 mM Tris, 1 mM EDTA). Final theoretical concentration is 25 ng/µl.

Stock the primers in aliquots. It is better to store stock solutions at -20°C, while working dilutions should be stored at 4°C

Prepare three independent reaction mixes for the three assays as follows: 0.2µM outer primers (F3 and B3), 1.6µM inner primers (FIP and BIP), 0.8uM loop primers (LF and LB), 1x buffer solution, 1.4mM dNTPs mix, 0.8M betaine, 8 U Bst Polymerase. Final volume of the reaction mix must be 25 µl, (23 µl reaction mix and 2 ul sample). Always keep reagents on ice. Prepare each of the the mixtures for at least 29 samples, comprising three 100%M, three 50%M, three 10%M, three 5%M, three 2.5%M, three 1%M, three 0.5%M, three 0.25%M, three 100%U and two no target controls for each assay.

Add 2µl (50 ng) of treated mixes of human genomic DNA to the reaction tubes, in triplicate for each DNA mix. Add 2ul of water in the no target controls samples, in duplicate. Close all the tubes.

### 2. Reaction

Program the turbidimeter in order to obtain a constant reaction temperature of 63°C for DAPK1 and GATA5 assays and of 65°C for CDKN2A assay for 1 hour (three distinct runs).

Put the reaction tubes in the instrument immediately before the beginning of the programs. Start the program.

### 3. Data analysis

Analyze the raw absorbance data using a customized MS Excel macro for baseline correction and threshold time calculation.

### Results

Selectivity of the three assays was evaluated, intended as the lowest methylated/unmethylated DNA ratio reproducibly detectable by each assay, i.e. the capability of each assay of discriminating the lowest dose possible of specific DNA in an aspecific background. Target mixtures containing decreasing amounts of methylated DNA (50%M, 10%M, 5%M, 2.5%M, 1 %M, 0.5%M, 0.25%M) were assayed with each LAMP assay along with positive (100%M) and negative controls (100%U and no target controls). Figure 5 shows amplification curves for each mixture down to the lowest reproducibly (3 repetitions) detected ratio which was 0.5%M for CDKN2A (figure 5A) and DAPK1 (figure 5B and 1%M for GATA5 (figure 5C). No aspecific amplification was obtained in NTC and 100% unmethylated DNA in 1 hour reaction.

### Example 4 Materials, methods and results of Multiplex MS-LAMP

### Reagents

### DAPK1 plasmids controls (synthesized by GeneArt): as in example 1

### CDKN2A plasmids controls (synthesized by GeneArt):

● Unmethylated plasmid (U), containing part of the expected sequence of the completely unmethylated human CDKN2A gene promoter after complete bisulfite conversion.
   Insert sequence (SEQ ID N.21): 
5'ATTAGAGGGTGGGGTGGATTGTGTGTGTTTGGTGGTTGTGGAGAGGGGGAG
AGTAGGTAGTGGGTGGTGGGGAGTAGTATGGAGTTGGTGGTGGGGAGTAGTA
TGGAGTTTTTGGTTGATTGGTTGGTTATGGTTGTGGTTTGGGGTTGGGTAGAGG
 
AGGTGTGGGTGTTGTTGGAGGTGGGGGTGTTGTTTAATGTATTGAATAGTTATG
GTTGGAGGTTGATTTAGG3’
● Methylated plasmid (M), containing part of the expected sequence of the fully methylated human CDNK2A gene promoter after complete bisulfite conversion. Insert sequence (SEQ ID N.22): 
5’ATTAGAGGGTGGGGCGGATCGCGTGCGTTCGGCGGTTGCGGAGAGGGGGA
GAGTAGGTAGCGGGCGGCGGGGAGTAGTATGGAGTCGGCGGCGGGGAGTAG
TATGGAGTTTTCGGTTGATTGGTTGGTTACGGTCGCGGTTCGGGGTCGGGTAG
AGGAGGTGCGGGCGTTGTTGGAGGCGGGGGCGTTGTTTAACGTATCGAATAG
TTACGGTCGGAGGTCGATTTAGG3’
 GATA5 plasmids controls (synthesized by GeneArt)
● Unmethylated plasmid (U), containing part of the expected sequence of the completely unmethylated human GATA5 gene promoter after complete bisulfite conversion.
   Insert sequence (SEQ ID N.23) 
5'TTTTTTTTTTGTGTTTGTTTTTTTTTTTTTGTAGGGTTAGTGTTGGGGTTTTGG
TTGTAGAGTTATGTGATTTTGGTAGGTTTTGTTTGTGGGGTTTGGTGATAAGGAT
GTATGATATGGGGTGGTTAGTGTGGAGTTTGGATTAGTGTAGTTAGATGGGTG
GGTTGAGGGTTTTGTGGGTTTTGGGGAGTTGGGTGTAGAGATTTTTTTAAGTTT
GTGTGAGTTTTGAGTTTTTTG3'
● Methylated plasmid (M), containing part of the expected sequence of the fully methylated human GATA5 gene promoter after complete bisulfite conversion.
   Insert sequence (SEQ ID N.24): 
5'TTTTTTTTTCGCGTTCGTTTTTTTTTTTCGTAGGGTTAGCGTTGGGGTTTCG
GTCGTAGAGTTACGTGATTTTGGTAGGTTTTGTTCGCGGGGTTTGGCGATAAG
GACGTACGATACGGGGCGGTTAGCGCGGAGTTCGGATTAGTGTAGTTAGACG
GGCGGGTCGAGGGTTTCGTGGGTTTCGGGGAGTCGGGCGTAGAGATTTTTTA
AGTTTGTGCGAGTTTCGAGTTTTTTG3'
Primers: synthesized by Eurofins MWG Operon, referred to as *"primers":*
DAPK1 PRIMER SET
As in **Example 1.**
CDKN2A PRIMER SET
As in **Example 2.**
GATA5 PRIMER SET
As in **Example 2.**
Reaction buffer: 200mM Tris HCl pH 8.8, 100mM KCl, 80mM MgSO4, 100mM (NH4)2SO4, 1% Tween, *"buffer 10x"*
dNTPs mix 25mM (Fermentas), *"dNTPs"*
Betaine 5M (Sigma Aldrich), *"betaine"*
Bst Large Fragment polymerase 8U/ul (New England Biolabs), *"Polymerase"*
Sterile apyrogen water (SALF Spa), *"ddw"*

### Material

Turbidimeter (Teramecs LA-200) for incubation at constant temperature and real time monitoring of turbidity

### Procedure

### Sample preparation

### Reaction

### Data Analysis

### 1. sample preparation

Stock the primers in aliquots. It is better to store stock solutions at -20°C, while working dilutions should be stored at 4°C

Prepare a triplex reaction mix containing all the 18 primers of the three assays as follows: 0.2µM of each outer primer (F3 and B3: FZ165, FZ166, FZ222, FZ223, FZ152, FZ147), 1.6µM of each inner primer (FIP and BIP: FZ176, FZ177, FZ224-t, FZ225-t, FZ157-T, FZ155-t), 0.8uM of each loop primer (LF and LB: FZ178, FZ179, FZ226,FZ227, FZ158, FZ151), 1x buffer solution, 1.4mM dNTPs mix, 0.8M betaine, 8 U Bst Polymerase. Final volume of the reaction mix must be 25 ul, (22 ul reaction mix and 3 µl sample). Always keep reagents on ice. Prepare the mix for at least ten samples, comprising 2 CDKN2A-GATA5-DAPK1 methylated sample, 2 CDKN2A-GATA5 methylated sample, 2 GATA5 methylated sample, 2 all unmethylated sample and 2 no target controls.

Add 1µl of each plasmid (15000 cps/µl) to the reaction tubes, in duplicate for each condition, following this scheme: two samples with 1 µl of each of the three plasmids in their methylated version, two samples with 1 µl of both CDKN2A and GATA5 in methylated version and 1 µl of DAPK1 plasmid in unmethylated version, two samples with 1 µl of GATA5 plasmid in methylated version and 1 µl of both CDKN2A and DAPK1 in unmethylated version, two samples with each of the three plasmids in their unmethylated version. Add 3ul of water in the no target controls samples, in duplicate. Close all the tubes.

### 2. Reaction

Program the turbidimeter in order to obtain a constant reaction temperature of 63°C for 1 hour.

Put the reaction tubes in the instrument immediately before the beginning of the programs. Start the program.

### 3. Data analysis

Analyze the raw absorbance data using a customized MS Excel macro for baseline correction and threshold time calculation.

### Results

All the 18 primers constituting the three LAMP assays were included in a single reaction to evaluate the capability of the triplex assay to detect at least one methylated target in a complex mixture. This triplex assay was used to test various mixtures of plasmid synthetic targets (figure 6), containing respectively three unmethylated targets, one methylated (GATA5) and two unmethylated (CDKN2A and DAPK1) targets, two methylated (GATA5 and CDKN2A) and one unmethylated (DAPK1) target or all the three methylated targets. A no target control was also included. DNA amplification occurred in presence of at least one methylated target. No amplification was obtained in the reaction containing only the unmethylated versions of the three plasmids at the same time or in no target controls.

### Example 5 Materials, methods and results of Fluorescent Multiplex MS-LAMP

### Reagents

DAPK1 plasmids controls (synthesized by GeneArt): as in example 1
CDKN2A plasmids controls (synthesized by GeneArt): as in example 4
GATA5 plasmids controls (synthesized by GeneArt) as in example 4
Primers: synthesized by Eurofins MWG Operon, referred to as *"primers":*
DAPK1 PRIMER SET
**FZ165** (F3) GGAGGATAGTCGGATCGAGTT (SEQ ID N.3)
**FZ166** (B3) CGCTATCGAAAACCGACCA (SEQ ID N.4)
**FZ176** (FIP) AACGCCGACCCCAAACCCTACGGATTTTGTTTTTTTCGCGGA (SEQ ID N.5)
**FZ177** (BIP) GGGAGGGATTTGCGTTTTTTATTCCAACGCCGAAAACTAACC (SEQ ID N.6)
**FZ178B** (LF) BodipyFL-CGCTACGAATTACCGAATCCC (SEQ ID N.25)
**FZ179** (LB) AGTTGTGTTTTCGTCGTCGTT (SEQ ID N.8)
CDKN2A PRIMER SET
**FZ222** (F3) TGGGGCGGATCGCGTG (SEQ ID N.9)
**FZ223** (B3) ACCGTAACTATTCGATACGTTA (SEQ ID N.10)
**FZ224-t** (FIP) CCCGCCGCCGACTCCATACTACTTCGGCGGTTGCGGA (SEQ ID N.11)
**FZ225-t** (BIP) GGAGTTTTCGGTTGATTGGTTGGTCCCCCGCCTCCAACAA (SEQ ID N.12)
**FZ226T** (LF) TAMRA-CGCTACCTACTCTCCCCCTC (SEQ ID N.26)
**FZ227** (LB) GGGTCGGGTAGAGGAGGTG (SEQ ID N. 14)
GATA5 PRIMER SET
**FZ152** (F3) TCGTAGGGTTAGCGTTGG (SEQ ID N.15)
**FZ147** (B3) CTCGAAACTCGCACAAAC (SEQ ID N.16)
**FZ157-t** (FIP) TAACCGCCCCGTATCGTACGTCGTCGTAGAGTTACGTGATTTTGG (SEQ ID N.17)
**FZ155-t** (BIP) GAGTTCGGATTAGTGTAGTTAGACGGTCTCTACGCCCGACTCC (SEQ ID N.18)
**FZ291B650** (LF) *Bodipy650*/*665-*CAAACCCCGCGAACAAAA (SEQ ID N.27)
**FZ151** (LB) GGTCGAGGGTTTCGTGGGT (SEQ ID N.20)
Reaction buffer: 200mM Tris HCl pH 8.8, 100mM KCl, 80mM MgSO4, 100mM (NH4)2SO4, 1% Tween, *"buffer 10x"*
dNTPs mix 25mM (Fermentas), *"dNTPs"*
Betaine 5M (Sigma Aldrich), *"betaine"*
Bst Large Fragment polymerase 8U/ul (New England Biolabs), *"Polymerase"*
Sterile apyrogen water (SALF Spa), *"ddw"*

### Material

Thermal cycler for incubation at constant temperature and real time monitoring of fluorescence at green, yellow and red channels.

### Procedure

### Sample preparation

### Reaction

### Data Analysis

### 1. sample preparation

Stock the primers in aliquots. It is better to store stock solutions at -20°C, while working dilutions should be stored at 4°C

Prepare a triplex reaction mix containing all the 18 primers of the three assays as follows: 0.2µM outer primers (F3 and B3), 1.6µM inner primers (FIP and BIP), 0.8uM loop primers and fluorescently marked oligonucleotides of both GATA5 and CDKN2A primer sets, 0.08 µM outer primers (F3 and B3), 0.64 µM inner primers (FIP and BIP), 0.32 uM loop primer and fluorescently marked primer of DAPK1 assay, 1x buffer solution, 1.4mM dNTPs mix, 0.8M betaine, 16 U Bst Polymerase. Final volume of the reaction mix must be 25 ul, (22 ul reaction mix and 3 µl sample). Always keep reagents on ice. Prepare the mix for at least ten samples, comprising 2 CDKN2A-GATA5-DAPK1 methylated sample, 2 CDKN2A methylated sample, 2 GATA5 methylated sample, 2 DAPK1 methylated sample and 2 all unmethylated samples.

Add 1µl of each plasmid (15000 cps/µl) to the reaction tubes, in duplicate for each condition, following this scheme: two samples with 1 µl of each of the three plasmids in their methylated version, two samples with 1 µl of CDKN2A in methylated version and 1 µl of DAPK1 and GATA5 plasmids in unmethylated version, two samples with 1 µl of GATA5 in methylated version and 1 µl of DAPK1 and CDKN2A plasmids in unmethylated version and two samples with 1 µl of DAPK1 in methylated version and 1 µl of CDKN2A and GATA5 plasmids in unmethylated version, two samples with each of the three plasmids in their unmethylated version. Close all the tubes.

### 2. Reaction

Program the thermal cycler in order to obtain a constant reaction temperature of 63°C for 1 hour.

Put the reaction tubes in the instrument immediately before the beginning of the programs. Start the program.

### 3. Data analysis

Analyze the raw fluorescence data using a customized MS Excel macro for baseline correction and threshold time calculation. Raw fluorescence data recorded during amplification are transformed first in percentage of the maximum fluorescence value reached in each reaction well and then expressed as percentage of quenching. Threshold-time values of each amplified sample are obtained by calculating the reaction time at which 50% of total quenching is reached.

### Results

In the triplex reaction one loop primer per assay was substituted by a differently labeled oligonucleotide (with the same DNA sequence as in CDKN2A and DAPK1 or slightly modified as in GATA5 assay) that allowed the real time detection of each of the three individual amplification events by simultaneously monitoring the quenching of fluorescence in the green, yellow and red channels of the instrument The fluorescence emitted by each dye is progressively quenched as the the primer which it is linked to, is progressively incorporated into the final amplification product. Real time amplification curves can be therefore depicted as percentage of the total quenching for each reaction. For ease in comprehension only one of the two repetitions for each reaction is showed in figure 7. In the three samples containing only one methylated plasmidic target (panels A, B, C), we could detect a quenching of the fluorescence only in the channel referring to the labelled oligonucleotide specific for that methylated plasmid. No amplification curve could be detected in any fluorescence channel if only unmethylated versions of all the plasmids were used as reaction targets (panel D), demonstrating that in absence of a methylated target no aspecific reaction product is generated. In the triplex reaction where all the three methylated target were present at the same time (panel E), all the three channels showed an amplification curve, demonstrating all that three different reactions were taking place simultaneously in the same reaction tube.

### Bibliography

1 Bestor TH. The DNA methyltransferases of mammals. Hum Mol Genet. 2000;9(16):2395-402.
2 Jones PA, Baylin SB. The epigenomics of cancer., Cell. 2007 Feb 23;128(4):683-92.
3 Egger G, Liang G, Aparicio A, Jones PA. Epigenetics in human disease and prospects for epigenetic therapy. Nature. 2004;429(6990):457-63
4 Maekawa M, Watanabe Y. Epigenetics: relations to disease and laboratory findings. Curr Med Chem. 2007;14(25):2642-53.
5 Costello JF, Plass C. Methylation matters. J Med Genet. 2001;38(5):285-303
6 Das PM, Singal R. DNA methylation and cancer. J Clin Oncol. 2004 Nov 15;22(22):4632-42.
7 Frommer M, McDonald LE, Millar DS, Collis CM, Watt F, Grigg GW, Molloy PL, Paul CL. A genomic sequencing protocol that yields a positive display of 5-methylcytosine residues in individual DNA strands. Proc Natl Acad Sci U S A. 1992;89(5):1827-31
8 Xiong Z, Laird PW. COBRA: a sensitive and quantitative DNA methylation assay. Nucleic Acids Res. 1997 Jun;25(12):2532-4
9 Herman JG, Graff JR, Myohanen S, Nelkin BD, Baylin SB. Methylation-specific PCR: a novel PCR assay for methylation status of CpG islands. Proc Natl Acad Sci U S A 1996;93:9821-6.
10 Eads CA, Danenberg KD, Kawakami K, Saltz LB, Blake C, Shibata D, Danenberg PV, Laird PW. MethyLight: a high-throughput assay to measure DNA methylation. Nucleic Acids Res. 2000;28(8):E32.

## Claims

1. A method for detecting patterns of methylations in at least one target nucleic acid sequence comprising a region with at least one CpG dinucleotide, comprising the steps of :
a. obtaining a nucleic acid sample comprising the at least one target nucleic acid sequence;
b. treating said nucleic acid sample with sodium bisulfite in appropriate conditions to obtain the conversion of cytosine residues into uracil residues except for methylated cytosines within a CpG dinucleotide sequence;
c. purifying the sodium bisulphate-treated nucleic acid;
d. contacting the purified nucleic acid sample, in appropriate reaction conditions, with a solution comprising a mixture of oligonucleotides and a DNA polymerase with strand displacement activity under isothermal hybridization conditions, wherein said mixture of oligonucleotides consists of primers suitable for a loop amplification mediated polymerization (LAMP) of the target nucleic acid sequence only if the conversion of step b. did not happen, wherein at least one of said primers suitable for LAMP comprises a region that is complementary to the region of the target nucleic acid sequence with at least one CpG dinucleotide, to obtain specific amplified products of the methylated target nucleic acid sequence;
e. detecting of said specific amplified products.

2. Method of claim 1 wherein all of said primers suitable for LAMP comprise a region that is complementary to the region of the target nucleic acid sequence with CpG dinucleotide,

3. Method of claim 1 or 2 wherein each CpG dinucleotide is not present within three nucleotides from the 3'-end of a F3, B3, F2, B2, LF or LB primer and within three nucleotides from the 5'-end of a F1 or B1 primer.

4. Method of any of previous claims wherein step e. is achieved by turbidity measurements following precipitation of insoluble pyrophosphate salts.

5. Method of claim 3 wherein the turbidity measurements are performed in real-time manner.

6. Method of claims 1 and 2 wherein step e. is achieved by fluorescence measurements.

7. Method of claim 5 wherein fluorescence measurements are performed in real-time manner.

8. Method of claims 6 or 7 wherein the fluorescent signal is generated by at least one fluorescently labelled oligonucleotide contained in the reaction mix

9. Method of claims 6 or 7 wherein the fluorescent signal is generated by an intercalating dye contained in the solution at item d. of claim 1.

10. Method of claims 6 or 7 wherein the fluorescent signal is generated by calcein contained in the solution at item d. of claim 1.

11. Method of any of previous claims wherein the target nucleic acid sequence belongs to a gene or to a regulative region thereof linked to a pathologic or pathology-prognostic status.

12. Method according to claim 11 wherein the gene belongs to the following group: ABCB1, APC, ARHI, BCL2, BRCA1, CALCA, CCND2, CDH1, CDH13, CDKN2A (p16), YP1B1, DAPK1, ESR1, ESR2, GATA1, GATA2, GATA3, GATA4, GATA5, GSTP1, HRAS1, HSD17B4, MCJ, MGMT, MLH1, MYOD1, Pax5a, PGR, PTGS2, RASSF1A, RNR1, SOCS1, SYK, TERT, TFF1, TGFBR2, THBS1, TIMP3, TNFRSF12, TWIST, TYMS, ACTB, ACTB, COL2A1, COL2A1.

13. Method of any of previous claims wherein LAMP primers are able of amplifying at least two different target nucleic acid sequences.

14. Method of claim 13 including LAMP primers are able of amplifying at least three different target nucleic acid sequences.

15. Method of claim 14 where the three different target nucleic acid sequences belong to the genes: CDKN2A, DAPK1, GATA5.

16. Method of claim 15 wherein primers are :
**FZ165** (F3) GGAGGATAGTCGGATCGAGTT (SEQ ID N.3);
**FZ166** (B3) CGCTATCGAAAACCGACCA (SEQ ID N.4);
**FZ176** (FIP) AACGCCGACCCCAAACCCTACGGATTTTGTTTTTTTCGCGGA (SEQ ID N.5);
**FZ177** (BIP) GGGAGGGATTTGCGTTTTTTATTCCAACGCCGAAAACTAACC (SEQ ID N.6):
**FZ178** (LF) CGCTACGAATTACCGAATCCC (SEQ ID N.7);
**FZ179** (LB) AGTTGTGTTTTCGTCGTCGTT (SEQ ID N.8);
**FZ222** (F3) TGGGGCGGATCGCGTG (SEQ ID N.9);
**FZ223** (B3) ACCGTAACTATTCGATACGTTA (SEQ ID N.10);
**FZ224-t** (FIP) CCCGCCGCCGACTCCATACTACTTCGGCGGTTGCGGA (SEQ ID N.11);
**FZ225-t** (BIP) GGAGTTTTCGGTTGATTGGTTGGTCCCCCGCCTCCAACAA (SEQ ID N.12);
**FZ226** (LF) CGCTACCTACTCTCCCCCTC (SEQ ID N.13);
**FZ227** (LB) GGGTCGGGTAGAGGAGGTG (SEQ ID N. 14);
**FZ152** (F3) TCGTAGGGTTAGCGTTGG (SEQ ID N.15);
**FZ147** (B3) CTCGAAACTCGCACAAAC (SEQ ID N.16);
**FZ157-t** (FIP) TAACCGCCCCGTATCGTACGTCGTCGTAGAGTTACGTGATTTTGG (SEQ ID N.17);
**FZ155-t** (BIP) GAGTTCGGATTAGTGTAGTTAGACGGTCTCTACGCCCGACTCC (SEQ ID N.18);
**FZ158** (LF) GCCAAACCCCGCGAACA (SEQ ID N.19);
**FZ151** (LB) GGTCGAGGGTTTCGTGGGT (SEQ ID N.20).

17. Method of claim 15 wherein primers are :
**FZ165** (F3) GGAGGATAGTCGGATCGAGTT (SEQ ID N.3);
**FZ166** (B3) CGCTATCGAAAACCGACCA (SEQ ID N.4);
**FZ176** (FIP) AACGCCGACCCCAAACCCTACGGATTTTGTTTTTTTCGCGGA (SEQ ID N.5);
**FZ177** (BIP) GGGAGGGATTTGCGTTTTTTATTCCAACGCCGAAAACTAACC (SEQ ID N.6);
**FZ178B** (LF) *BodipyFL*-CGCTACGAATTACCGAATCCC (SEQ ID N.25);
**FZ179** (LB) AGTTGTGTTTTCGTCGTCGTT (SEQ ID N.8);
**FZ222** (F3) TGGGGCGGATCGCGTG (SEQ ID N.9);
**FZ223** (B3) ACCGTAACTATTCGATACGTTA (SEQ ID N.10);
**FZ224-t** (FIP) CCCGCCGCCGACTCCATACTACTTCGGCGGTTGCGGA (SEQ ID N.11);
**FZ225-t** (BIP) GGAGTTTTCGGTTGATTGGTTGGTCCCCCGCCTCCAACAA (SEQ ID N.12);
**FZ226T** (LF) TAMRA-CGCTACCTACTCTCCCCCTC (SEQ ID N.26);
**FZ227** (LB) GGGTCGGGTAGAGGAGGTG (SEQ ID N. 14);
**FZ152** (F3) TCGTAGGGTTAGCGTTGG (SEQ ID N.15);
**FZ147** (B3) CTCGAAACTCGCACAAAC (SEQ ID N.16);
**FZ157-t** (FIP) TAACCGCCCCGTATCGTACGTCGTCGTAGAGTTACGTGATTTTGG (SEQ ID N.17);
**FZ155-t** (BIP) GAGTTCGGATTAGTGTAGTTAGACGGTCTCTACGCCCGACTCC (SEQ ID N.18);
**FZ291B650** (LF) *Bodipy650*/*665-*CAAACCCCGCGAACAAAA (SEQ ID N.27);
**FZ151** (LB) GGTCGAGGGTTTCGTGGGT (SEQ ID N.20).
